# EUROPÄISCHE PATENTSCHRIFT

(11) **EP 2 623 293 B1**
(45) Veröffentlichungstag und Bekanntmachung des Hinweises auf die Patenterteilung: **01.06.2016**
(21) Anmeldenummer: 13163477.6
(22) Anmeldetag: 31.07.2009
(51) Int. Cl.: B29C 49/42, B29C 49/46

(54) **Vorrichtung und Verfahren zum Sterilisieren von Kunststoffvorformlingen**
Method and device for sterilising plastic pre-forms
Dispositif et procédé de stérilisation d'ébauches en plastique

(30) Priorität: 18.08.2008 DE 102008038143
(43) Veröffentlichungstag der Anmeldung: 07.08.2013
(62) Teilanmeldung aus: 09781331.5
(73) Patentinhaber: Krones AG, 93073 Neutraubling (DE)
(72) Erfinder: Engelhard, Patrick, 93073 Neutraubling (DE); Dahmen, Michael, 93073 Neutraubling (DE); Martini, Oliver, 93073 Neutraubling (DE)
(74) Vertreter: Bittner, Bernhard

(56) Entgegenhaltungen:
- EP-A1- 1 122 168
- FR-A1- 2 774 912
- FR-A1- 2 887 525
- FR-A1- 2 907 684
- US-A1- 2004 113 328
- DATABASE WPI Week 199206 Thomson Scientific, London, GB; AN 1992-045642 XP002699581, -& JP H03 290226 A (DAINIPPON PRINTING CO LTD) 19. Dezember 1991 (1991-12-19)

## Beschreibung

Die vorliegende Erfindung bezieht sich auf eine Vorrichtung zum Herstellen von Kunststoffbehältnissen. Derartige Vorrichtungen sind aus dem Stand der Technik seit langem bekannt. Dabei werden üblicherweise Kunststoffvorformlinge zunächst erwärmt und anschließend mittels einer Umformungseinrichtung, wie insbesondere einer Blaseinrichtung, zu Kunststoffbehältnissen expandiert. Bei vielen Anwendungen ist es dabei erforderlich, eine Sterilisation der Kunststoffbehältnisse vorzunehmen, insbesondere bei sensiblen Getränken wie beispielsweise Fruchtsäften oder Milch oder auch bei Medikamenten.

Üblicherweise wird im Stand der Technik eine Sterilisation der fertig geblasenen Behältnisse durchgeführt, wobei insbesondere eine Innenwandung dieser Behältnisse sterilisiert wird. Der Nachteil dieser Sterilisation der Behältnisse besteht darin, dass diese bereits eine relativ große Fläche aufweisen und daher eine große Menge an Sterilisationsmittel, wie beispielsweise Wasserstoffperoxidgas, nötig ist.

Daher ist man im Stand der Technik teilweise dazu übergegangen, nicht die Kunststoffbehältnisse, sondern die Kunststoffvorformlinge zu sterilisieren, da diese eine wesentlich geringere Innenoberfläche aufweisen als die fertig geblasenen Behältnisse.

Aus der EP 1 086 019 B1 ist ein Verfahren und eine Vorrichtung zum Herstellen steriler Verpackungsbehälter bekannt. Dabei wird bei einem entsprechenden Verfahren zunächst eine Vorheizstufe vorgesehen, in der das expandierbare Material auf eine Innenflächentemperatur erhitzt wird, welche eine Taupunkttemperatur eines gasförmigen Sterilisierungswirkstoffes übersteigt, jedoch unter der Glasierungstemperatur des Thermoplastmaterials liegt. Weiterhin ist eine Sterilisierungsstufe vorgesehen, in der der Sterilisierungswirkstoff in der Gasphase eingeführt wird sowie eine Endheizstufe, in der der Zuschnitt auf eine Temperatur oberhalb der Glasierungstemperatur des Zuschnitts erhitzt wird. Damit erfolgt hier der Sterilisationsvorgang innerhalb einer Heizeinrichtung, welche die Vorformlinge erwärmt damit diese anschließend mittels Blasluft erhitzt werden können. Auf diese Weise kann die Temperatur einer Heizeinrichtung gleichzeitig verwendet werden, um den Sterilisationsprozess zu erleichtem. Andererseits besteht jedoch auch der Nachteil, dass die entsprechende Heizeinrichtung durch das Sterilisationsmittel, wie beispielsweise Wasserstoffperoxid, auf Dauer beschädigt werden kann.

Aus der EP 0 996 530 B1 ist ein Verfahren zum Herstellen eines sterilen Behälters ausgehend von einer Vorform bekannt. Dabei wird ein Sterilisierprodukt, das durch Wärme aktivierbar ist, auf eine Vorform aufgebracht und die Vorform erwärmt, um gleichzeitig das Stertlisierprodukt zu aktivieren. Auch bei diesem Verfahren besteht jedoch der Nachteil, das die Sterilisation in einer Heizeinrichtung durchgeführt wird, die an sich dem Vorerwärmen der Vorformlinge dient und die daher durch das oftmals sehr aggressive Gas angegriffen werden kann.

Aus der FR 2887525 ist eine Vorrichtung zum Herstellen sterilisierter Flaschen bekannt, welche eine Sterilisationseinheit aufweist. Die US 2004/0113328 beschreibt biaxial orientierte thermoplastische Hohlkörper und verbesserte Sterilisationsverfahren. Die FR 2907684 beschreibt ein Verfahren zum Sterilisieren von Vorformlingen sowie einen Ofen zu deren Erwärmung.

Aus der EP 1122168 sind ein Verfahren zum Formen und Füllen von Behältnissen bekannt. Dabei werden in einem Schritt Vorformlinge sterilisiert. Die FR 2774912 offenbart ebenfalls ein Verfahren zum Sterilisieren von Behältnissen, wobei ein Sterilisationsmittel auch auf eine Innenwandung der Behältnisse aufgebracht wird.

Weiterhin sind aus dem Stand der Technik diverse Vorrichtungen bekannt, welche die Behältnisse ohne Verwendung von gasförmigen Medien sterilisieren, beispielsweise unter Verwendung von Elektronenstrahlen oder UV-Licht. Derartige Vorrichtungen bedürfen jedoch oftmals einer sehr genauen Abschirmung, damit die teilweise sehr gefährlichen Strahlen nicht nach außen treten können.

Der vorliegenden Erfindung liegt daher die Aufgabe zugrunde, eine Vorrichtung zum Herstel len von Kunststotfibehältnissen zur Verfügung zu stellen, welche eine kostengünstige und effiziente Sterilisation der Behältnisse ermöglicht. Dies wird erfindungsgemäß durch eine Vorrichtung nach Anspruch 1 und ein Verfahren nach Anspruch 9 erreicht. Vorteilhafte Ausführungsformen und Weiterbildungen sind Gegenstand der Unteransprüche.

Eine erfindungsgemäße Vorrichtung zum Herstellen von Kunsistoffhahältnissen weist eine Heizeinrichtung auf, welche Kunststoffvorformlinge erwärmt, sowie eine Transporteinrichtung, welche die Kunststoffvorformlinge entlang eines vorgegebenen Transportpfads transportiert und eine bezüglich der Heizeinrichtung entlang des Transportpfads stromabwärts angeordneten Umformungseinrichtung, welche die Kunststoffvorformlinge zu Kunststoffbehältnissen umformt.

Erfindungsgemäß weist die Vorrichtung eine Sterilisationseinrichtung auf, welche ein fließfähiges Medium auf eine Wandung und insbesondere eine Innenwandung bzw. einen Bereich dieser Innenwandung der Kunststoffvorformlinge aufbringt um diese Innenwandung zu sterilisieren, wobei diese Sterilisationseinrichtung derart bezüglich des Transportpfades der Behältnisse angeordnet ist, dass die Sterilisation nach dem Ende des Heizvorgangs der Behältnisse in der Heizeinrichtung und vor dem Ende des Umformungsvorganges in der Umformungseinrichtung stattfindet.

Durch die erfindungsgemäße Vorrichtung wird einerseits gewährleistet, dass die Sterilisation nicht erst an dem fertig hergestellten Behältniss stattfindet sondern bevorzugt noch in einem Zustand, in dem das Behältniss noch als Vorformling vorliegt. Im Gegensatz zu dem oben zitierten Stand der Technik findet die Sterilisation nach dem Heizvorgang der Vorformlinge statt.

Die Erfindung beruht damit auf dem Gedanken, dass einerseits noch die Wärme ausgenutzt wird, da die Vorformlinge selbst schon auf eine bestimmte Wärme aufgewärmt wurden andererseits jedoch der Sterilisationsvorgang nicht in der Heizvorrichtung selbst vorgenommen wird und damit auch Beschädigungen der Heizvorrichtung durch das fließfähige Medium verhindert werden. Bei dem fließfähigen Medium handelt es sich um ein gasförmiges Medium, welches auf die Innenwandung der Kunststoffvorformlinge aufgebracht wird.

Bevorzugt ist jedoch die Sterilisationsvorrichtung auf dem Transportpfad zwischen der Heizeinrichtung und der Umformungseinrichtung angeordnet. Damit ist bevorzugt die Sterilisationseinrichtung als Modul vorgesehen, welches zwischen der Heizeinrichtung und der Umformungseinrichtung vorgesehen ist. Es wäre jedoch auch möglich, die Sterilisationseinrichtung noch in der Heizeinrichtung, jedoch auf dem Transportpfad nach einem letzten Heizelement dieser Heizeinrichtung anzuordnen. Auch wäre es möglich, die Sterilisationseinrichtung an den Anfang der Umformungseinrichtung zu stellen oder in die Umformungseinrichtung zu integrieren.

So wäre es beispielsweise auch möglich, den Expansionsvorgang auch mit einem Sterilisationsmedium wie beispielsweise Wasserstoffperoxidgas durchzuführen. Dabei könnte beispielsweise dieses Sterilisationsgas durch eine hohle Reckstange in das Innere der Vorformlinge eingeführt werden. Auch könnte ein Teil des Expansionsvorgangs der Behältnisse mit dem Sterilisationsgas durchgeführt werden. Bevorzugt jedoch ist die Sterilisation der Innenwandung der Behältnisse bereits abgeschlossen, wenn die Kunststoffvorformlinge die Umformungseinrichtung erreichen.

Bei einer weiteren vorteilhaften Ausführungsform ist die Sterilisationseinrichtung an einer zwischen der Heizeinrichtung und der Umformungseinrichtung vorgesehenen Transporteinheit vorgesehen. Vorzugsweise weist eine gesamte Transporteinrichtung eine Vielzahl von Einzelelementen wie Transportsterne, Übergabesterne und dergleichen auf, wobei die Sterilisationseinrichtung auf einem insoweit eigenen Transportstern oder Transportrad angeordnet sein kann.

Vorzugsweise weist die Sterilisationseinrichtung eine Vielzahl von Düseneinrichtungen auf, welche in das Innere der Kunststoffvorformlinge eingeführt werden können und somit zumindest abschnittsweise mit den Behältnissen entlang deren Transportpfad mitgeführt werden.

Bei einer weiteren vorteilhaften Ausführungsform weist die Vorrichtung eine Erwärmungseinheit auf, welche das fließfähige Medium vor dem Aufbringen auf die Innenwandung der Vorformlinge erwärmt. Dabei ist es möglich, dass der Vorformling zunächst in die Heizeinrichtung bzw. in einen Ofen gefahren wird und auf eine Verformungstemperatur aufgeheizt wird. Dabei wird die höchste Temperatur des Vorformlings erreicht. Anschließend wird ein Desinfektionsmittel, wie ein oxidierendes Desinfektionsagens in einem Sterilisationsmodul nach dem Ofen in den Vorformling eingeblasen.

Die Temperatur dieses gasförmigen Wasserstoffperoxids ist dabei vorzugsweise bereits über dem Aktivierungsniveau dieses Gases, wobei zum Erwärmen des Gases vorzugsweise die erwähnte Erwärmungseinheit eingesetzt wird. Eine Kondensation des gasförmigen Wasserstoffperoxid bzw. Desinfektionsagens an der Innenwandung des Vorformlings wird aufgrund der hohen Temperatur des Vorformlings verhindert, da der Taupunkt des Wasserstoffperoxids stets niedriger ist als die Verformungstemperatur.

Bei einer weiteren vorteilhaften Ausführungsform sind wenigstens die Sterilisationseinrichtung und die Umformungseinrichtung in einem Reinraum angeordnet. Es wäre jedoch auch möglich, beispielsweise auch die Heizeinrichtung in dem besagten Reinraum anzuordnen. Weiterhin könnte ein Reinraum auch einen nachfolgenden Bereich der Vorrichtung, wie beispielsweise einen Verschließer für die Behältnisse, umfassen.

Bei einer weiteren vorteilhaften Ausführungsform weist die Vorrichtung eine weitere Sterilisationseinrichtung auf, welche eine Aussenwandung der Kunststoffvorformlinge sterilisiert. Diese weitere Sterilisationseinheit kann dabei bevorzugt nach der besagten ersten Sterilisationseinheit angeordnet werden und beispielsweise in einem Einlauf der Umformungseinrichtung vorgesehen sein. Bei dieser zweiten Sterilisationseinheit handelt sich bevorzugt ebenfalls um eine Sterilisationseinheit, welche ein gasförmiges Medium wie Wasserstoffperoxid zur Sterilisation verwendet. Es könnten jedoch auch andere Sterilisationseinheiten vorgesehen sein und insbesondere solche, welche zum Sterilisieren elektromagnetische Strahlung einsetzen, wie insbesondere aber nicht ausschließlich UV-Licht und / oder Elektronenstrahlen.

Bei einer weiteren vorteilhaften Ausführungsform weist die Vorrichtung eine Ausblaseeinheit auf, welche die Kunststoffvorformlinge bzw. deren Innenwandung nach der Sterilisation mit einem gasförmigen Medium, bevorzugt mit Sterilluft, ausbläst. Diese Ausblaseinheit gewährleistet, dass Reste des Wasserstoffperoxidgases weitgehend vollständig aus dem Inneren des Vorformlings ausgeblasen werden. Andererseits kann es jedoch auch bewusst in Kauf genommen werden, das ein gewisser Rest an Desinfektionsmittel bzw. Wasserstoffperoxidgas in die Umformungseinheit transportiert wird, damit beispielsweise auch Blasformhälften dieser Umformungseinheiten mit sterilisiert werden.

Vorzugsweise handelt es sich bei dem fließfähigen Medium um Wasserstoffperoxid.

Die vorliegende Erfindung ist weiterhin auf ein Verfahren zum Herstellen von Kunststoffbehältnissen gerichtet, wobei in einem ersten Verfahrensschritt Kunststoffvorformlinge entlang eines vorgegebenen Transportpfades transportiert werden. Weiterhin werden die Kunststoffvorformling während deren Transport entlang des Transportpfades mittels einer Heizeinrichtung erwärmt und anschließend werden die Kunststoffvorformlinge mittels einer Umformungseinrichtung zu Kunststoffbehältnissen umgeformt wobei die Umformungseinrichtung auf dem Transportpfad stromabwärts bezüglich der Heizeinrichtung angeordnet ist. Erfindungsgemäß wird eine Wandung und bevorzugt eine Innenwandung der Kunststoffvorformlinge nach Beendigung des Erwärmungsvorgangs und vor Beendigung des Umformungsvorganges mittels eines fleißfähigen Mediums sterilisiert. Vorzugsweise wird die Innenwandung nach Beendigung des Erwärmungsvorgangs und vor Beginn des Umformungsvorgangs sterilisiert.

Bevorzugt erfolgt die Sterilisation der Innenwandung durch Einblasen des fließfähigen Mediums in die Kunststoffvorformlinge.

Vorteilhaft wird das fließfähige Medium vor dem Einblasen in die Kunststoffvorformlinge erwärmt und insbesondere bereits vor dem Einblasen in die Kunststoffvorformlinge über seine Aktivierungstemperatur erwärmt. Es wäre jedoch auch möglich, dass das fließfähige Medium vor dem Einblasen seine Aktivierungstemperatur noch nicht erreicht hat und diese erst innerhalb der Vorformlinge erreicht, da die Innentemperatur der Vorformlinge erheblich über dieser Aktivierungstemperatur liegt.

Bei einem weiteren bevorzugten Verfahren ist während des Einblasens des fließfähigen Mediums in die Behältnisse eine Temperatur der Innenwandung der Kunststoffvorformlinge höher als sein Taupunkt des fließfähigen Mediums.

Weitere Vorteile und Ausführungsformen ergeben sich aus der beigefügten Zeichnung. Dabei zeigt:

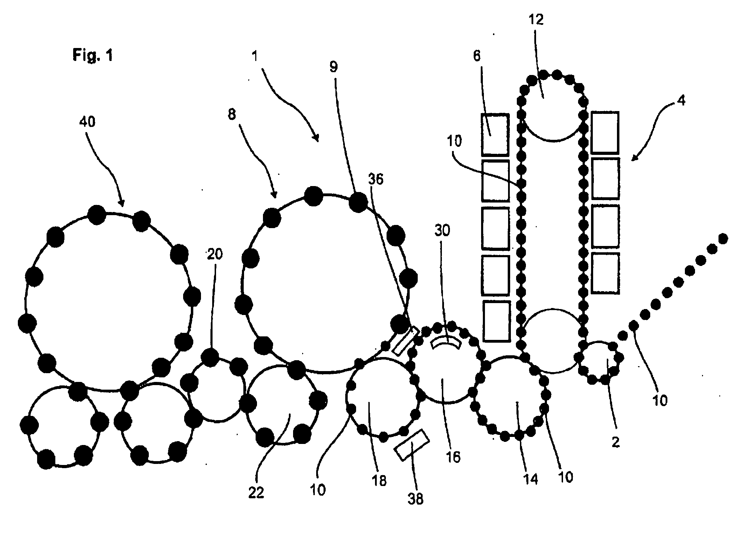

- Fig. 1: eine schematische Darstellung einer erfindungsgemäßen Vorrichtung;

Fig. 1 zeigt eine grob schematische Darstellung einer erfindungsgemäßen Vorrichtung 1.

Diese Vorrichtung 1 weist eine Zuführeinrichtung 2 auf, welche Vorformlinge 10 getaktet bzw. mit exakter Teilung einer Heizeinrichtung 4 zuführt. Diese Heizeinrichtung 4 weist dabei eine Vielzahl von Heizelementen 6 auf, die je entlang der Längsseiten der Heizeinrichtung jeweils außerhalb der Vorformlinge 10 angeordnet ist. Das Bezugszeichen 12 bezieht sich auf eine Transporteinrichtung wie je eine umlaufende Transportkette, welche die Behältnisse 10 durch die Heizeinrichtung 4 transportiert. An die Heizeinrichtung 4 schließt sich eine Transporteinrichtung 14 bzw. eine Teileinheit 14 der Transporteinrichtung an. Bei dieser Transporteinrichtung 14 handelt es sich um einen so genannten Teilungsverzugsstern, mit dem beispielsweise die Teilung zwischen Vorformlingen 10 geändert werden kann. Das Bezugszeichen T bezieht sich auf einen Transportpfad, entlang dessen die Kunststoffvorformlinge 10 bzw. anschließend die Kunststoffbehältnisse 20 transportiert werden.

An diese Transporteinrichtung 14 schließt sich eine weitere Transporteinrichtung 16 an, wobei im Bereich dieser Tranpsorteinrichtung 16 eine Sterilisation der Vorformlinge 10 mit Hilfe einer Sterilisationseinrichtung 30 stattfindet. Diese Sterilisationseinrichtung 30 ist hier nur schematisch gezeigt. Vorzugsweise werden jedoch Elemente dieser Sterilisationseinrichtung 30 mit den Vorformlingen 10 mitgeführt, das heißt im Bereich der Transporteinrichtung 16 mit den Vorformlingen mitgeführt. Bevorzugt weist die Sterilisationseinrichtung 30 eine Vielzahl von Füllelementen auf, welche in die Mündungen der Vorformlinge einführbar sind, um diese mit dem Desinfektionsagens zu beaufschlagen. Dabei sind bevorzugt diese Füllelemente in einer Längsrichtung der Kunststoffvorformlinge 10 bewegbar. Es wäre jedoch auch möglich, die Kunststoffvorformlinge selbst in deren Längsrichtung zu bewegen.

An die Sterilisationseinrichtung 30 bzw. die Transporteinrichtung 16 schließt sich eine weitere Transporteinrichtung 18 an, welche hier ebenfalls wieder als Teilungsverzugsstern ausgeführt ist, der die Teilung der Kunststoffvorformlinge ändern kann. Dieser Teilungsverzugsstern 18 übergibt die Behältnisse an eine Umformungseinrichtung 8, die hier eine Vielzahl von Blasstationen 9 aufweist, welche zum Expandieren der Kunststoffvorformlinge mittels Druckluft zu Kunststoffbehältnissen 20 dient. Die in ihrer Gesamt nicht mit einem Bezugszeichen versehene Transporteinrichtung weist eine Vielzahl von Transporteinheiten 12, 14, 16, 18 auf.

Das Bezugszeichen 36 bezieht sich auf eine weitere Sterilisationseinrichtung, welche insbesondere aber nicht ausschließlich eine Aussenwandung der Kunststoffvorformlinge 10 sterilisliert. Daneben kann eine weitere Sterilisationseinrichtung 38 auch stationär in dem Bereich der Transporteinrichtung 16 vorgesehen sein.

An die Umformungseinrichtung 8 schließt sich eine weitere Transporteinrichtung 22 an, und an diese letztlich eine Fülleinrichtung 40, welche die umgeformten Kunststoffbehältnisse mit einem Medium, wie beispielsweise einem Getränk, befüllt.

Obwohl nicht gezeigt, kann die Vorrichtung wie oben erwähnt eine Vielzahl von weiteren Sterilisationseinrichtungen aufweisen, welche beispielsweise in der Heizeinrichtung, an der Umformungseinrichtung oder auch dieser nachgeordnet sind. Weiterhin kann sich die gesamte Vorrichtung in einem Reinraum befinden wo auch möglicherweise innerhalb dieses Reinraums ein weiterer Reinraum in dem Bereich vorgesehen ist, der mit der Sterilisationseinrichtung 30 beginnt, wobei innerhalb dieses weiteren Reinraums bevorzugt ein höhrerer Reinheitsgrad bestehen kann als in dem erstgenannten Reinraum.

## Patentansprüche

1. Vorrichtung (1) zum Herstellen von Kunststoffbehältnissen (20), mit einer Heizeinrichtung (4), welche Kunststoffvorformlinge erwärmt, mit einer Transporteinrichtung (12, 14, 16, 18), welche die Kunststoffvorformlinge (10) entlang eines vorgegebenen Transportpfads (T) transportiert und mit einer bezüglich der Heizeinrichtung (4) entlang des Transportpfads stromabwärts angeordneten Umformungseinrichtung (8), welche die Kunststoffvorformlinge zu Kunststoffbehältnissen umformt,
wobei
die Vorrichtung (1) eine Sterilisationseinrichtung (30) aufweist, welche ein fliessfähiges Medium auf eine Wandung der Kunststoffvorformlinge (10) aufbringt, um diese Wandung zu sterilisieren, wobei diese Sterilisationseinrichtung derart bezüglich des Transportpfads der Behältnisse angeordnet ist, dass die Sterilisation nach dem Ende des Heizvorgangs der Behältnisse in der Heizeinrichtung (4) und vor dem Ende des Umformungsvorgangs in der Umformungseinrichtung stattfindet und wobei die Vorrichtung (1) eine Vielzahl von Düseneinrichtungen aufweist, welche hintereinander angeordnet sind und auf diese Weise gleichzeitig mehrere Vorformlinge mit Sterilisationsgas befüllen können,
wobei
die Sterilisationseinrichtung (30) an einer zwischen der Heizeinrichtung (4) und der Umformungseinrichtung (8) vorgesehenen Transporteinheit (16) vorgesehen ist **dadurch gekennzeichnet, dass** sich an die Steritisationseinrichtung (30) eine als Teilungsverzugsstem ausgeführte Transporteinrichtung (18) anschließt, durch welchen die Teilung der Kunststoffvorformlinge (10) veränderbar ist.

2. Vorrichtung (1) nach Anspruch 1, **dadurch gekennzeichnet, dass** die Sterilisationseinrichtung auf dem Transportpfad zwischen der Heizeinrichtung (4) und der Umformungseinrichtung (8) angeordnet ist.

3. Vorrichtung (1) nach wenigstens einem der vorangegangenen Ansprüche, **dadurch gekennzeichnet, dass** die Vorrichtung (1) eine Erwärmungseinheit aufweist, welche das fliessfähige Medium vor dem Aufbringen auf die Innenwandung der Vorformlinge (10) erwärmt.

4. Vorrichtung (1) nach wenigstens einem der vorangegangenen Ansprüche, **dadurch gekennzeichnet, dass** wenigstens die Sterilisationseinrichtung (30) und die Umformungseinrichtung (8) in einem Reinraum (32) angeordnet sind.

5. Vorrichtung (1) nach wenigstens einem der vorangegangenen Ansprüche, **dadurch gekennzeichnet, dass** die Vorrichtung (1) eine Ausblaseinheit aufweist, weiche die Kunststoffvorformlinge nach der Sterilisation deren Innenwandung mit einem gasförmigen Medium ausbläst.

6. Vorrichtung (1) nach wenigstens einem der vorangegangenen Ansprüche, **dadurch gekennzeichnet; dass** das fliessfähige Medium Wasserstoffperoxid ist.

7. Vorrichtung (1) nach wenigstens einem der vorangegangenen Ansprüche,
**dadurch gekennzeichnet, dass**
die gesamte Transporteinrichtung eine Vielzahl von Einzelelementen wie Transportsterne, Übergabesterne und dergleichen aufweist, und die Sterilisationseinrichtung auf einem eigenen Transportstem oder Transportrad angeordnet ist.

8. Vorrichtung (1) nach wenigstens einem der vorangegangenen Ansprüche,
**dadurch gekennzeichnet, dass**
die Sterilisationseinrichtung als Modul vorgesehen ist, welches zwischen der Heizenrichtung und der Umformungseinrichtung vorgesehen ist.

9. Verfahren zum Herstellen von Kunststoffbehältnissen (20) mit den Schritten:
- Transportieren von Kunststoffvorformlingen (10) entlang eines vorgegebenen Transportpfads (T) ;
- Erwärmen der Kunststoffvorformlinge (10) während deren Transport entlang des Transportpfads mittels einer Heizeinrichtung (4);
- Umformen der Kunststoffvorformlinge (10) zu Kunststoffbehältnissen (20) mittels einer Umformungseinrichtung (8), welche auf dem Transportpfad (T) stromabwärts bezüglich der Heizeinrichtung (4) angeordnet ist, wobei eine Wandung der Kunststoffvorformlinge (10) nach Beendigung des Erwärmungsvorgangs und vor Beendigung des Umformungsvorgangs mittels eines fliessfähigen Mediums sterilisiert wird, wobei die Sterilisationseinrichtung (30) an einer zwischen der Heizeinrichtung (4) und der Umformungseinrichtung (8) vorgesehenen Transporteinheit (16) vorgesehen ist **dadurch gekennzeichnet, dass** an die Sterilisationseinrichtung (30) eine als Teilung sverzugsstern ausgeführt Transporteinrichtung (18) angeschlossen ist, durch welchen die Teilung der Kunststoffvoriormlinge (10) verändert wird.

10. Verfahren nach Anspruch 9, **dadurch gekennzeichnet, dass** die Sterilisation der Innenwandung durch Einblasen des fliessfähigen Mediums in die Kunststoffvorformlinge erfolgt.

11. Verfahren nach wenigstens einem der vorangegangenen Ansprüche 9-10, **dadurch gekennzeichnet, dass** das fliessfähige Medium vor dem Einblasen in die Kunststoffvorformlinge erwärmt wird.

12. Verfahren nach wenigstens einem der vorangegangenen Ansprüche 10 - 11 **dadurch gekennzeichnet, dass** während des Einblasens des fliessfähigen Mediums in die Behältnisse eine Temperatur der Innenwandung der Kunststoffvorformlinge (10) höher ist als ein Taupunkt des fliessfähigen Mediums.

13. Verfahren nach wenigstens einem der vorangegangenen Ansprüche,
**dadurch gekennzeichnet, dass**
eine Temperatur des Desinfektionsmittels bereits vor dem Einblasen des Desinfektionsmittel in die Kunststoffvorformlinge über dem Aktivierungsniveau dieses Gases liegt.

14. Verfahren nach wenigstens einem der vorangegangenen Ansprüche,
**dadurch gekennzeichnet, dass**
Elemente der Sterilisationseinrichtung (30) mit den Vorformlingen 10 mitgeführt werden.

## Claims

1. An apparatus (1) for producing plastics material containers (20), with a heating device (4) which heats plastics material pre-forms, with a conveying device (12, 14, 16, 18) which conveys the plastics material pre-forms (10) along a pre-set conveying path (T), and with a shaping device (8) which is arranged downstream with respect to the heating device (4) along the conveying path and which shapes the plastics material pre-forms to form plastics material containers, wherein the apparatus (1) has a sterilization device (30) which applies a flowable medium to a wall of the plastics material pre-forms (10) in order to sterilize this wall, wherein this sterilization device is arranged with respect to the conveying path of the containers in such a way that the sterilization takes place after the end of the heating procedure of the containers in the heating device (4) and before the end of the shaping procedure in the shaping device and wherein the apparatus (1) comprises a plurality of nozzle devices which are arranged in series and in this way can fill several preforms with sterilization gas at the same time, wherein the sterilization device (30) is provided at a transport unit (16) provided between the heating device (4) and the shaping device (8), **characterized in that** a transport device (18) realized as a pitch changing star follows the sterilization device (30), by means of which the pitch of the plastics material preforms (10) can be changed.

2. An apparatus (1) according to claim 1, **characterized in that** the sterilization device is situated on the conveying path between the heating device (4) and the shaping device (8).

3. An apparatus (1) according to at least one of the preceding claims, **characterized in that** the apparatus (1) has a heating unit which heats the flowable medium before application to the inner wall of the pre-forms (10).

4. An apparatus (1) according to at least one of the preceding claims, **characterized in that** at least the sterilization device (30) and the shaping device (8) are arranged in a clean room (32).

5. An apparatus (1) according to at least one of the preceding claims, **characterized in that** the apparatus (1) has a blow-off unit which blasts the plastics material pre-forms with a gaseous medium after the sterilization of the inner wall thereof.

6. An apparatus (1) according to at least one of the preceding claims, **characterized in that** the flowable medium is hydrogen peroxide.

7. Apparatus (1) according to at least one of the preceding claims,
**characterized in that**
the whole transport device comprises a plurality of single elements, such as transport stars, pitch changing stars or the like and that the sterilization device is arranged on its own transport star or transport wheel.

8. Apparatus (1) according to at least one of the preceding claims,
**characterized in that**
the sterilization device is provided as a module, which is provided between the heating device and the shaping device.

9. A method of producing plastics material containers (20), with the steps:
- conveying plastics material pre-forms (10) along a pre-set conveying path (T);
- heating the plastics material pre-forms (10) during their transport along the conveying path by means of a heating device (4);
- shaping the plastics material pre-forms (10) to form plastics material containers (20) by means of a shaping device (8) which is arranged on the conveying path (T) downstream of the heating device (4), a wall of the plastics material pre-forms (10) being sterilized by means of a flowable medium after the end of the heating procedure and before the end of the shaping procedure, wherein the sterilization device (30) is provided at a transport unit (16) which is provided between the heating device (4) and the shaping device (8), **characterized in that** a transport device (18) realized as a pitch changing star is connected to the sterilization device (30), by means of which the pitch of the plastics material preforms (10) is changed.

10. A method according to claim 9, **characterized in that** the sterilization of the inner wall is carried out by blowing the flowable medium into the plastics material pre-forms.

11. A method according to at least one of the preceding claims 9 to 10, **characterized in that** the flowable medium is heated before being blown into the plastics material pre-forms.

12. A method according to at least one of the preceding claims 10 to 11, **characterized in that** while the flowable medium is blown into the containers a temperature of the inner wall of the plastics material pre-forms (10) is higher than a dew point of the flowable medium.

13. A method according to at least one of the preceding claims,
**characterized in that**
a temperature of the disinfection means is already above the activation level of this gas before the disinfection means is blown into the plastics material preforms.

14. A method according to at least one of the preceding claims,
**characterized in that**
elements of the sterilization device (30) are entrained with the preforms (10).

## Revendications

1. Installation (1) pour la fabrication de récipients en matière plastique (20), avec un dispositif de chauffage (4) chauffant des préformes en matière plastique, avec un dispositif de transport (12, 14, 16, 18) convoyant les préformes en matière plastique (10) le long d'un chemin de transport (T) défini et avec un dispositif de transformation (8) situé en aval du dispositif de chauffage (4) le long du chemin de transport et transformant les préformes en matière plastique en récipients en matière plastique,
ladite installation (1) comportant un dispositif de stérilisation (30) appliquant un milieu fluide sur une paroi des préformes en matière plastique (10) pour stériliser ladite paroi, ledit dispositif de stérilisation étant disposé par rapport au chemin de transport des récipients de telle manière que la stérilisation a lieu à l'issue du processus de chauffage des récipients dans le dispositif de chauffage (4) et avant la fin du processus de transformation dans le dispositif de transformation, et ladite installation (1) comportant une pluralité de dispositifs de buse situés les uns derrière les autres, plusieurs préformes pouvant ainsi être simultanément remplies d'un gaz de stérilisation,
le dispositif de stérilisation (30) étant prévu sur une unité de transport (16) prévue entre le dispositif de chauffage (4) et le dispositif de transformation (8),
**caractérisée en ce**
**qu'**un dispositif de transport (18) réalisé comme étoile réductrice permettant de modifier la répartition des préformes en matière plastique (10) est adjacent au dispositif de stérilisation (30).

2. Installation (1) selon la revendication 1, **caractérisée en ce que** le dispositif de stérilisation est situé entre le dispositif de chauffage (4) et le dispositif de transformation (8) sur le chemin de transport.

3. Installation (1) selon au moins une des revendications précédentes, **caractérisée en ce que** ladite installation (1) comporte une unité chauffante qui chauffe le milieu fluide avant son application contre la paroi intérieure des préformes (10).

4. Installation (1) selon au moins une des revendications précédentes, **caractérisée en ce qu'**au moins le dispositif de stérilisation (30) et le dispositif de transformation (8) sont situés dans un compartiment stérile (32).

5. Installation (1) selon au moins une des revendications précédentes, **caractérisée en ce que** ladite installation (1) comporte une unité de soufflage qui souffle un fluide gazeux dans les préformes en matière plastique après la stérilisation de leur paroi intérieure.

6. Installation (1) selon au moins une des revendications précédentes, **caractérisée en ce que** le milieu fluide est du peroxyde d'hydrogène.

7. Installation (1) selon au moins une des revendications précédentes,
**caractérisée en ce que**
l'ensemble du dispositif de transport comprend une pluralité d'éléments individuels tels qu'étoiles de transport, étoiles de transfert et similaires, et **en ce que** le dispositif de stérilisation est disposé sur une étoile de transport ou une roue de transport propre.

8. Installation (1) selon au moins une des revendications précédentes,
**caractérisée en ce que**
le dispositif de stérilisation est prévu comme module situé entre le dispositif de chauffage et le dispositif de transformation.

9. Procédé de fabrication de récipients en matière plastique (20), comprenant les étapes suivantes :
- transport de préformes en matière plastique (10) le long d'un chemin de transport (T) défini ;
- chauffage des préformes en matière plastique (10) pendant leur transport le long du chemin de transport au moyen d'un dispositif de chauffage (4) ;
- transformation des préformes en matière plastique (10) en récipients en matière plastique (20) au moyen d'un dispositif de transformation (8) situé en aval du dispositif de chauffage (4) sur le chemin de transport (T), une paroi des préformes en matière plastique (10) étant stérilisée au moyen d'un milieu fluide à l'issue du processus de chauffage et avant la fin du processus de transformation, le dispositif de stérilisation (30) étant prévu sur une unité de transport (16) prévue entre le dispositif de chauffage (4) et le dispositif de transformation (8),
**caractérisé en ce que**
le dispositif de stérilisation (30) est adjacent à un dispositif de transport (18) réalisé comme étoile réductrice permettant de modifier la répartition des préformes en matière plastique (10).

10. Procédé selon la revendication 9, **caractérisé en ce que** la stérilisation de la paroi intérieure est exécutée par soufflage du milieu fluide dans les préformes en matière plastique.

11. Procédé selon au moins une des revendications 9 et 10, **caractérisé en ce que** le milieu fluide est chauffé avant le soufflage dans les préformes en matière plastique.

12. Procédé selon au moins une des revendications 10 et 11, **caractérisé en ce que**, pendant le soufflage du milieu fluide dans les récipients, une température de la paroi intérieure des préformes en matière plastique (10) est supérieure à un point de rosée du milieu fluide.

13. Procédé selon au moins une des revendications précédentes,
**caractérisé en ce**
**qu'**avant même le soufflage de l'agent désinfectant dans les préformes en matière plastique, une température de l'agent désinfectant est supérieure au niveau d'activation dudit gaz.

14. Procédé selon au moins une des revendications précédentes,
**caractérisé en ce que**
des éléments du dispositif de stérilisation (30) sont entraînés avec les préformes (10).
